# FASCICULE DE BREVET EUROPEEN

(11) **EP 3 153 214 B1**
(45) Date de publication et mention de la délivrance du brevet: **04.03.2020**
(21) Numéro de dépôt: 16192684.5
(22) Date de dépôt: 06.10.2016
(51) Int. Cl.: A61Q 19/00, A61K 8/92, A61K 8/06, A61K 8/19, A61P 17/00

(54) **COMPOSITION À BASE D'HUILE VÉGÉTALE NATURELLE ET D'EAU DE CHAUX**
ZUSAMMENSETZUNG AUF DER BASIS VON NATÜRLICHEM PFLANZENÖL UND KALKWASSER
NATURAL VEGETABLE OIL AND LIME WATER-BASED COMPOSITION

(30) Priorité: 08.10.2015 FR 1559590
(43) Date de publication de la demande: 12.04.2017
(73) Titulaire: Laboratoires Chemineau, 37210 Vouvray (FR)
(72) Inventeur: AGNUS, Benoit, 37110 villedomer (FR); SACRE, Morgan, 37210 VOUVRAY (FR)
(74) Mandataire: Plasseraud IP

(56) Documents cités:
- Anonymous: "Comment Faire, Stabiliser & Epaissir du liniment Oleo-calcaire - By Reo - Cosmetiques Maison - DIY - Conseils Beauté, Maquillage, Savons & Soins Naturels BIO -", , 21 janvier 2009 (2009-01-21), XP055279727, Extrait de l'Internet: URL:http://byreo.canalblog.com/archives/20 09/01/21/12162384.html [extrait le 2016-06-10]
- Anonymous: "Liniment oléo-calcaire fait maison Recette | Chocolate & Zucchini", , 23 octobre 2014 (2014-10-23), XP055279720, Extrait de l'Internet: URL:http://chocolateandzucchini.com/vf/rec ettes/autres-recettes/liniment-oleo-calcai re-fait-maison-recette/ [extrait le 2016-06-10]
- Anonymous: "Le Liniment : recette facile et douce pour Bébé", , 12 juillet 2012 (2012-07-12), XP055279723, Extrait de l'Internet: URL:http://www.lessentieldejulien.com/2012 /07/le-liniment-une-recette-facile-et-douc e-pour-bebe/ [extrait le 2016-06-10]
- Anonymous: "Comment faire un liniment oléo calcaire onctueux et stabilisé", , 16 avril 2014 (2014-04-16), XP055279731, Extrait de l'Internet: URL:http://www.petitdeviendragrand.fr/comm ent-faire-un-liniment-oleo-calcaire-onctue ux-et-stabilise/ [extrait le 2016-06-10]
- Anonymous: "Créer vos produits naturels : simple et pas cher !: Trucs et astuces pour un liniment réussit !", , 9 juin 2011 (2011-06-09), XP055279733, Extrait de l'Internet: URL:http://sophieaunaturel.blogspot.de/201 1/06/trucs-et-astuces-pour-un-liniment.htm l [extrait le 2016-06-10]
- Gattefossé: "3 Waxes, 3 Benefits, 1 Ingredient acticire", , 16 avril 2013 (2013-04-16), XP055279702, Extrait de l'Internet: URL:http://www.scsformulate.co.uk/wp-conte nt/uploads/2015/08/Acticire-Brochure.pdf [extrait le 2016-06-10]

## Description

La présente invention a pour objet une composition naturelle à base d'huile végétale naturelle, d'eau de chaux, de cire d'abeille naturelle et d'au moins une cire végétale naturelle, ladite composition étant destinée à une application topique dans le domaine de la pharmacie et/ou de la cosmétologie.

Une composition à base d'huile d'olive et d'eau de chaux (l'eau de chaux est une solution d'hydroxyde de calcium) est connue dans l'état de l'art, et est plus couramment dénommée « Liniment calcaire ». La Pharmacopée Française la décrit comme suit :
- Solution d'hydroxyde de calcium : 50% (agent neutralisant),
- Huile d'olive vierge : 50% (adoucissant).

Une telle composition est principalement utilisée pour nettoyer la peau et prévenir ses irritations, et notamment l'érythème fessier du nourrisson. En effet, l'utilisation continue de couches, ainsi que l'acidité et la macération de l'urine peuvent devenir très irritantes pour la peau fragile des fesses du bébé. En appliquant une telle composition lors du change du bébé, les fesses sont nettoyées et un mince film lipidique recouvre la peau des fesses du bébé. Ceci permet d'isoler la peau de l'acidité des urines. D'autre part, le pH basique de la composition permet de contrebalancer l'acidité de l'urine et de restaurer le pH naturel de la peau. Ainsi la peau du bébé est protégée contre les irritations, et notamment l'érythème fessier.

Une telle composition a un aspect liquide huileux jaunâtre, et n'est pas stable dans le temps. Une agitation avant chaque utilisation est nécessaire pour homogénéiser la composition qui présente deux phases après un temps de repos (relargage d'huile en surface).

Les solutions au problème de stabilité préconisées dans l'état de l'art sont très souvent l'ajout d'un stabilisateur qui n'est pas naturel.

Il a également été préconisé dans l'art antérieur d'ajouter de la cire d'abeille, d'une part pour faciliter l'émulsion entre la phase aqueuse et la phase grasse, et d'autre part pour permettre une meilleure stabilisation de ces deux phases au repos.

L'ajout de cire d'abeille dans une composition à base d'huile d'olive et d'eau de chaux, à une concentration pouvant aller jusqu'à 8% par rapport au poids total de la composition, permet de faciliter la préparation de l'émulsion mais également d'améliorer sa stabilité à court terme.

Pour obtenir une composition stabilisée sur le long terme, l'ajout d'une quantité plus importante de cire d'abeille (à savoir supérieure à 8% par rapport au poids total de la composition) est nécessaire mais aura pour inconvénient d'épaissir significativement la composition et ainsi de rendre son application plus difficile et moins douce.

L'ajout de cire d'abeille seule à une concentration plus faible (à savoir inférieure ou égale à 8% par rapport au poids total de la composition), dans une composition à base d'huile d'olive et d'eau de chaux, n'est pas suffisante pour obtenir une composition à la fois fluide et stabilisée.

Ainsi, aucune des solutions préconisées dans l'art antérieur ne permet à ce jour d'obtenir à la fois une composition fluide, stabilisée de façon naturelle et de manière durable dans le temps.

De nombreuses recherches ont été menées par les Inventeurs afin de proposer une composition entièrement naturelle, qui soit fluide, stabilisée naturellement et de façon durable dans le temps, et se rapprochant le plus possible de la composition telle qu'elle est décrite dans la Pharmacopée Française.

Les Inventeurs ont ainsi découvert de manière surprenante et inattendue que l'ajout d'une quantité définie d'au moins une cire végétale naturelle ou d'un dérivé de cire(s) végétale(s) dans une composition à base d'huile d'olive, d'eau de chaux et de cire d'abeille permettait d'obtenir une composition fluide et stabilisée de façon naturelle et durable dans le temps.

Les Inventeurs ont également découvert qu'une telle composition ainsi stabilisée pouvait avantageusement être utilisée dans une composition pour une application topique.

La présente invention a ainsi pour objet une composition, caractérisée en ce qu'elle comprend :
- 40% à 54%, de préférence 44% à 50%, et plus préférentiellement encore 46% à 48% d'une huile végétale choisie dans le groupe comprenant l'huile d'olive, l'huile de macadamia, l'huile d'amande douce, l'huile de lin, l'huile d'abricot, l'huile d'argan, l'huile d'avocat, l'huile d'argousier, l'huile de germe de blé, l'huile de jojoba, l'huile de karité, l'huile d'onagre, l'huile de pépin de raisin, l'huile de noisette, l'huile de sésame, l'huile de soja, l'huile de tournesol, l'huile de bourrache et leurs mélanges, et de préférence l'huile d'olive,
- 40% à 60%, de préférence 45% à 55%, et plus préférentiellement encore 48% à 52% d'une solution d'hydroxyde calcium,
- 0,5% à 8%, de préférence 1% à 6%, et plus préférentiellement encore 1,5% à 2,5% de cire d'abeille Cera Alba,
- 0,1 à 1,2% d'une cire végétale choisie parmi :
   - un dérivé de cires obtenu par trans-estérification de la cire de jojoba, de la cire de tournesol et de la cire de mimosa, ou
   - un mélange d'ester de jojoba, de cire de tournesol et de cire de mimosa,
les pourcentages étant en poids par rapport au poids total de la composition.

L'huile végétale, la cire d'abeille, la cire végétale et le dérivé de cire(s) végétale(s) sont naturels et sont de préférence issus de l'agriculture biologique.

Un composé naturel ou une composition naturelle est un composé ou une composition constitué(e) uniquement d'ingrédients d'origine naturelle.

Selon un mode de réalisation de l'invention, la cire végétale de la composition de l'invention est un mélange de cire de jojoba, de cire de tournesol et de cire de mimosa.

On entend par « dérivé de cire(s) végétale(s) », un produit issu de la transformation d'une cire végétale ou d'un mélange de cires végétales, notamment par estérification ou trans-estérification.

Un exemple de dérivé de cire végétale est l'ester de jojoba.

Un dérivé de cires végétales utilisé selon l'invention est le dérivé de cires obtenu par trans-estérification de la cire de jojoba, de la cire de tournesol et de la cire de mimosa.

Selon encore un autre mode de réalisation de l'invention, le dérivé de cires végétales de la composition de l'invention est un mélange d'ester de jojoba, de cire de tournesol et de cire de mimosa.

A titre d'exemple, un tel mélange de cires pourra comprendre :
- 50% à 75% d'ester de jojoba ou de cire de jojoba, de préférence de 58% à 67%,
- 25% à 50% de cire de tournesol, de préférence de 33% à 42%,
- 1% à 5% de cire de mimosa, de préférence de 2% à 4%,
les pourcentages étant en poids par rapport au poids total du complexe.

Il sera également possible d'ajouter un autre composé gras naturel, comme par exemple un triglycérol, à ladite cire végétale ou audit dérivé de cire(s) végétale(s).

La composition de l'invention est particulièrement avantageuse puisque l'association de cire d'abeille avec une cire végétale ou le dérivé de cire(s) végétale(s) tels que définis ci-dessus, permet d'obtenir une composition fluide et stabilisée de façon naturelle dans le temps, sans aucune séparation des différents constituants de ladite composition.

Selon l'invention, on entend par composition « stable » ou « stabilisée » une composition qui ne présente pas de déphasage visible à l'œil nu.

La composition de l'invention est donc encore caractérisée en ce qu'elle ne comprend pas d'agent stabilisateur tels que ceux habituellement utilisés dans les domaines de la pharmacie et/ou de la cosmétologie.

Selon un mode de réalisation de l'invention, l'huile végétale est de l'huile d'olive.

Selon un autre mode de réalisation de l'invention, l'huile végétale est un mélange d'huile d'olive et d'une autre huile végétale choisie dans le groupe comprenant l'huile de macadamia, l'huile d'amande douce, l'huile de lin, l'huile d'abricot, l'huile d'argan, l'huile d'avocat, l'huile d'argousier, l'huile de germe de blé, l'huile de jojoba, l'huile de karité, l'huile d'onagre, l'huile de pépin de raisin, l'huile de noisette, l'huile de sésame, l'huile de soja, l'huile de tournesol, l'huile de bourrache et leurs mélanges.

Selon l'invention, lorsque l'huile végétale est un mélange d'huile d'olive avec une autre huile, alors le ratio « huile d'olive/autre huile végétale » est de 99,9/0,1 à 40/60 en poids par rapport au poids total du mélange « huile d'olive et autre huile végétale ».

La présente invention a également pour objet une composition telle que définie ci-dessus, caractérisée en ce qu'elle comprend en outre :
- un extrait de plante, notamment choisi dans le groupe comprenant un extrait de plante de la famille des Malvaceae, à titre d'exemple la guimauve ou la mauve, de camomille, d'aloe vera, d'euphraise, d'ortie, de sauge, de souci, de pivot, de pivoine, de mélèze, de pensée, de patience, de pâquerette, de pissenlit, de ronce, de souci, de saponaire, de molène, de fleur de Calendula, et leurs mélanges,
- un extrait de fruit, notamment choisi dans le groupe comprenant un extrait de canneberge, de cassis, de myrtille, de grenade, de framboise, de mûre, de figuier, de prunelle et leurs mélanges,
- une huile essentielle, notamment choisie dans le groupe comprenant l'huile essentielle de bergamote, de rose, d'orange douce, de lavandin, de cyprès, de niaouli, de romarin, de sauge, de géranium, de thym, d'eucalyptus globulus, de menthe, de matricaire, de genévrier, de colchique, de pivot d'Amérique, de vanille et leurs mélanges,
- de l'oxyde de zinc, du bicarbonate de soude, du silicate de magnésium et/ou du D-panthénol,
- un agent de restructuration cellulaire, de préférence l'acide hyaluronique,
- un prébiotique, notamment choisi dans le groupe comprenant des glucooligosaccharides, des fructooligosaccharides et leurs mélanges,
- un probiotique, de préférence les Lactobacillus,
- un agent antimicrobien,
- un agent antifongique, et/ou
- un parfum naturel.

Selon un mode de réalisation de l'invention, dans la composition telle que définie ci-dessus :
- l'extrait de plante est présent en une quantité en poids allant de 0% à 1,5%, de préférence de 0,1% à 1%,
- l'extrait de fruit est présent en une quantité en poids allant de 0% à 2%, de préférence de 0,5% à 1,5%,
- l'extrait d'huile essentielle est présent en une quantité en poids allant de 0% à 1,5%, de préférence de 0,1% à 1%,
- l'oxyde de zinc, le bicarbonate de soude, le silicate de magnésium et/ou le D-panthénol est (sont) présent(s) en une quantité en poids allant de 0% à 5%, de préférence de 0,1% à 3%,
- l'agent de restructuration cellulaire est présent en une quantité en poids allant de 0% à 0,3%, de préférence de 0,03 à 0,1%,
- le prébiotique est présent en une quantité en poids allant de 0 à 5%, de préférence de 0,5 à 3%,
- l'agent antimicrobien et/ou l'agent antifongique est (sont) présent(s) en une quantité en poids allant de 0% à 3%, de préférence de 0,1% à 2%, et plus préférentiellement encore de 0,3 à 1%,
- le parfum est présent une quantité en poids allant de 0% à 0,5%, de préférence de 0,01% à 0,1%, et plus préférentiellement encore de 0,03 à 0,07%,
chacune desdites quantités en poids étant définie par rapport au poids total de la composition.

Selon un autre mode de réalisation de l'invention, dans la composition telle que définie ci-dessus, le probiotique est présent en une quantité de 150 millions à 15 milliards, de préférence de 1 milliard à 5 milliards d'unités vivantes.

Selon un mode de réalisation avantageux de l'invention, les compositions de l'invention présentent une stabilité, lorsqu'elles sont placées dans une enceinte climatique à une température de 40°C et à un taux d'humidité relative de 75%, supérieure à 1 mois, de préférence supérieure à 2 mois, et plus préférentiellement encore supérieure à 3 mois.

L'invention a également pour objet l'utilisation d'une composition telle que définie ci-dessus dans une composition pour une application topique.

Dans ce qui suit, afin de distinguer les deux compositions de la présente demande, la composition pour une application topique telle que définie dans le paragraphe ci-dessus sera désignée par composition « complexe » par opposition à la composition telle que définie ci-dessus qui elle sera désignée par composition « simple » ou « composition telle que définie ci-dessus ».

Le Demandeur précise également que la composition « simple » est également appropriée pour une application topique.

L'invention a encore pour objet une composition pour une application topique (composition « complexe »), caractérisée en ce qu'elle comprend :
- une composition telle que définie ci-dessus (composition « simple »),
- un composé choisi dans le groupe comprenant un agent émollient, un agent humectant, un agent émulsifiant, une gomme et leurs mélanges.

Plus particulièrement, selon un mode de réalisation avantageux de l'invention, dans la composition complexe :
- l'agent émollient est choisi dans le groupe comprenant le beurre, l'huile de karité, le cholestérol, le beurre de cacao, un triglycéride d'acide caprylique /caprique, et leurs mélanges,
- l'agent humectant est choisi dans le groupe comprenant le xylitol, le sorbitol, le glycérol, le pentylène glycol, le sodium hyaluronate et leurs mélanges, et est de préférence le xylitol,
- l'agent émulsifiant est choisi dans le groupe comprenant un ester de sorbitane, le monostéarate de glycérol, la lécithine de soja, un mélange d'un alcool en C₁₄₋₂₂ et d'un glucoside d'alkyle en C₁₂₋₂₀, un mélange d'un alcool cétéarylique et de cétéaryl glucoside, un mélange d'un alcool arachidylique, d'un alcool béhénylique et d'un arachydil glucoside, et leurs mélanges,
- la gomme est choisie dans le groupe comprenant la gomme d'acacia, la gomme xanthane, la gomme de guar, la gomme de biosaccharide et leurs mélanges.

Selon un autre mode de réalisation de l'invention, dans la composition complexe telle que définie ci-dessus :
- la composition telle que définie ci-dessus (la composition « simple ») est présente en une quantité en poids allant de 1% à 95%,
- le composé choisi dans le groupe comprenant un agent émollient, un agent humectant, un agent émulsifiant, une gomme et leurs mélanges est présent en une quantité en poids allant de 5% à 99%,
chacune desdites quantités en poids étant définie par rapport au poids total de la composition.

La présente invention a également pour objet l'utilisation d'une composition « simple » ou « complexe » telle que définie ci-dessus, sous forme de crème, pommade, mousse, cérat ou baume.

Un autre objet de l'invention porte aussi sur l'utilisation d'une composition « simple » ou « complexe » telle que définie ci-dessus, pour nettoyer et/ou hydrater la peau.

De manière générale, les compositions de l'invention pourront être utilisées pour simplement nettoyer, hydrater et/ou protéger la peau.

Cependant, elles pourront également être utilisées pour cicatriser, réparer et/ou apaiser des peaux irritées. A titre d'exemple d'irritation de la peau, on pourra notamment citer l'érythème fessier du nourrisson.

La présente invention a encore pour objet une composition « simple » ou « complexe » telle que définie ci-dessus, pour une utilisation dans la cicatrisation, la réparation et/ou l'apaisement de la peau.

Les propriétés des compositions de l'invention pourront varier en fonction de l'huile végétale spécifiquement choisie, et l'ajout ou non d'ingrédients décrits comme optionnels dans la composition simple ou complexe de la présente invention.

De façon générale, tout extrait de plante ou de fruit ainsi que toute huile essentielle traditionnellement reconnus pour leurs propriétés apaisantes, émollientes, cicatrisantes et anti-inflammatoires pourront être ajoutés dans les compositions de l'invention.

Les compositions simples ou complexes de l'invention pourront être conditionnées dans les conditionnements habituellement utilisés pour les applications topiques. A titre d'exemple on pourra citer une bouteille, un tube, munis ou non d'un système de dispensation, un système airless, un boîtier aérosol avec une valve classique ou dite à poche, munie d'un diffuseur (le diffuseur est positionné sur la valve afin de permettre la sortie du produit par pression manuelle sur celui-ci), une unidose ou un sachet.

Les exemples suivants illustrent l'invention, ils ne la limitent en aucune façon.
La **figure 1** est un comparatif de la stabilité entre des compositions « simples » de l'invention (à savoir les compositions 1, 7 et 12 décrites dans l'exemple 1 ci-dessous) et quatre compositions de l'art antérieur (compositions A, B, C et D), stockées dans une enceinte climatique, à une température de 40°C et à un taux d'humidité relative de 75%, après 1 mois de stockage (**figure 1a**) après 12 mois de stockage (**figure 1b**).
La **figure 2** est un comparatif de l'aspect microscopique de la composition 1 de l'invention (**figure 2a**) et de deux compositions de l'art antérieur, C (**figure 2b**) et D (**figure 2c**).
La **figure 3** est un comparatif du profil rhéologique de la composition 1 de l'invention (**figure 3a**) et de deux compositions de l'art antérieur, C (**figure 3b**) et D (**figure 3c**).

### Exemple 1

Toutes les compositions décrites dans cet exemple ont été préparées sous forme de crème. Elles correspondent à des compositions « simples ».

Dans les tableaux 1 à 3 ci-après sont décrits les différents constituants des compositions simples de l'invention, avec leurs pourcentages en poids respectifs (lesdits pourcentages étant définis par rapport au poids total de la composition).

Le dérivé de cires végétales naturelles utilisé dans cet exemple est un mélange d'ester de jojoba, de cire de tournesol et de cire de mimosa.

La dénomination INCI de ce dérivé est plus particulièrement la suivante : « Jojoba Esters (et) Helianthus Annuus Seed Wax (et) Acacia Decurrens Flower Wax (et) Polyglycerin-3 ». A titre indicatif, ce complexe est commercialisé sous la dénomination Acticire ®.

Les compositions simples 1, 2, 4 et 6 de l'invention sont décrites dans le tableau 1 (les compositions 3 et 5 sont hors invention):

| **Composition** | % (m/m) **Huile d'olive vierge** | % (m/m) **Eau de chaux** | % (m/m) **Cire d'abeille (Cera Alba)** | % (m/m) **Acticire ®** |
|---|---|---|---|---|
| 1 | 47 | 50 | 2 | 1 |
| 2 | 40 | 57 | 2 | 1 |
| 3 | 43 | 54 | 0,5 | 2,5 |
| 4 | 54 | 40 | 5 | 1 |
| 5 | 45 | 49 | 2 | 4 |
| 6 | 45 | 46,90 | 8 | 0,1 |

Les compositions simples 7 à 11 de l'invention sont décrites dans le tableau 2 :

| **Composition** | % (m/m) **Huile d'olive vierge** | % (m/m) **Autre huile végétale** | % (m/m) **Eau de chaux** | % (m/m) **Cire d'abeille (Cera Alba)** | % (m/m) **Acticire ®** |
|---|---|---|---|---|---|
| 7 | 18,80 | **Huile de tournesol** 28,20 | 50 | 2 | 1 |
| 8 | 42 | **Huile de germe de blé** 5 | 50 | 2 | 1 |
| 9 | 44 | **Huile d'amande douce** 3 | 50 | 2 | 1 |
| 10 | 43 | **Huile de macadamia** 4 | 50 | 2 | 1 |
| 11 | 46 | **Huile d'avocat** 1 | 50 | 2 | 1 |

Les compositions simples 12 à 21 de l'invention sont décrites dans le tableau 3 :

| **Composition** | % (m/m) **Huile d'olive vierge** | % (m/m) **Eau de chaux** | % (m/m) **Cire d'abeille (Cera Alba)** | % (m/m) **Acticire ®** | % (m/m) **Autre** |
|---|---|---|---|---|---|
| 12 | 47 | 49,50 | 2 | 1 | **Extrait de camomille** 0,5 |
| 13 | 47 | 49,50 | 2 | 1 | **Extrait de mauve** 0,5 |
| 14 | 47 | 49,50 | 2 | 1 | **Extrait de prunelle** 0,5 |
| 15 | 47 | 49,50 | 2 | 1 | **Extrait d'Aloe vera** 0,5 |
| 16 | 47 | 49,50 | 2 | 1 | **Extrait de canneberge** 0,5 |
| 17 | 47 | 49,50 | 2 | 1 | **Extrait d'euphraise** 0,5 |
| 18 | 47 | 49,80 | 2 | 1 | **HE d'orange douce** 0,2 |
| 19 | 47 | 49,80 | 2 | 1 | **HE de bergamote** 0,2 |
| 20 | 45,50 | 48,50 | 2 | 1 | **Oxyde de zinc** 1 |
| 21 | 47 | 49,50 | 2 | 1 | **Dexpanthenol** 1 |

| | | | | | |
|---|---|---|---|---|---|
| HE : huile essentielle | | | | | |

### Exemple 2 : stabilité des compositions simples de l'invention

Des essais de stabilité des compositions décrites dans les tableaux 1 à 3 ci-dessus ont été conduits.

Toutes les compositions se sont avérées stables à température ambiante allant de 15 à 20°C, pendant une période de 9 mois.

En effet, aucun déphasage n'est observé dans les compositions de l'invention après un stockage de 9 mois.

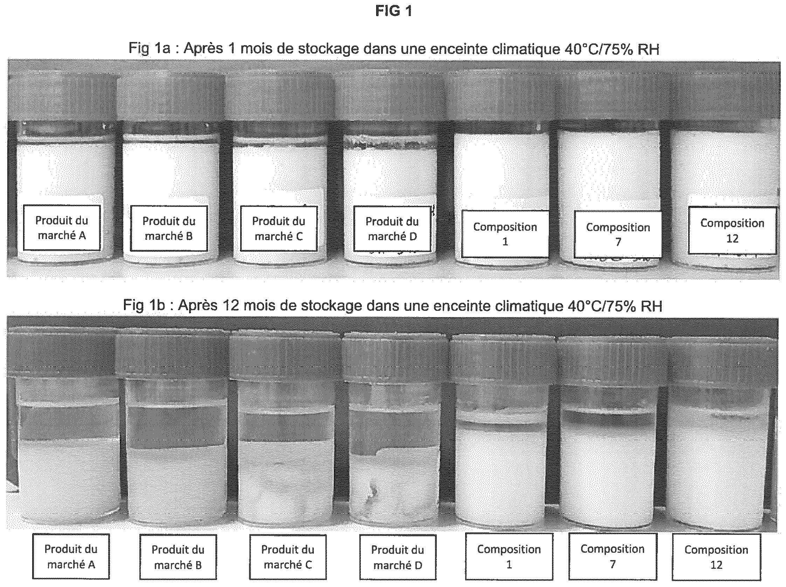

Par ailleurs, la stabilité des compositions 1, 7 et 12 de l'invention a été comparée à celle de quatre compositions de l'art antérieur (A à D) trouvées sur le marché (**figure 1**), dans des conditions drastiques, à savoir un placement des différentes compositions en étuves à 40°C et à un taux d'humidité résiduelle de 75 %.

La composition des quatre produits A à D de l'art antérieur est respectivement la suivante :

### Composition A :

Huile d'olive,
Hydroxyde de calcium,
Cire d'abeille,
Stéarate de glycéryle, Pentylène glycol, Caprylyl glycol, Décylène glycol, Eau.

### Composition B :

Huile d'olive,
Hydroxyde de calcium,
Stéaralkonium Hectorite, Caprylyl glycol, Carbonate de propylène, Ethyl héxyl glycérine, polymère d'oxyde d'éthylène éthoxylé, Vitamine E, Palmitate d'ascorbyle, Acide ascorbique, Acide critique, Eau.

### Composition C :

Huile d'olive,
Hydroxyde de calcium,
Stéarate de glycéryle, Eau.

### Composition D :

Huile d'olive,
Hydroxyde de calcium,
Caprylyl glycol, Carbomer, Hydroxyde de sodium, Oléate de sorbitan, Extrait de carotte, Huile de maïs, Vitamine E, Eau.

### Résultats

Après 1 mois de stockage en étuves (**fig. 1a**), le relargage d'huile en surface (ou déphasage) est observable sur les compositions de l'art antérieur, et ceci même pour la composition A de l'art antérieur contenant de la cire d'abeille (pourtant connue pour aider à la stabilisation d'une telle composition), contrairement aux compositions de l'invention pour lesquelles aucun relargage n'est observé.

Après douze mois (**fig. 1b**) le relargage (déphasage) est très important pour les produits du marché quand celui des compositions de l'invention reste tout à fait raisonnable au vu des conditions drastiques de stockage.

En outre, après 12 mois, une simple agitation des compositions de l'invention permet instantanément de retrouver une émulsion homogène, contrairement aux compostions de l'art antérieur qui mettent beaucoup plus de temps à retrouver un aspect homogène.

### Exemple 3

L'aspect microscopique de la composition 1 de l'invention a été comparé à celui des compositions C et D de l'art antérieur, décrites dans l'exemple 2 ci-dessus.

### Appareillage utilisé :

Scope A1 de chez Zeiss.
Conditions opératoires :
Zoom x20, grossissement x1000.

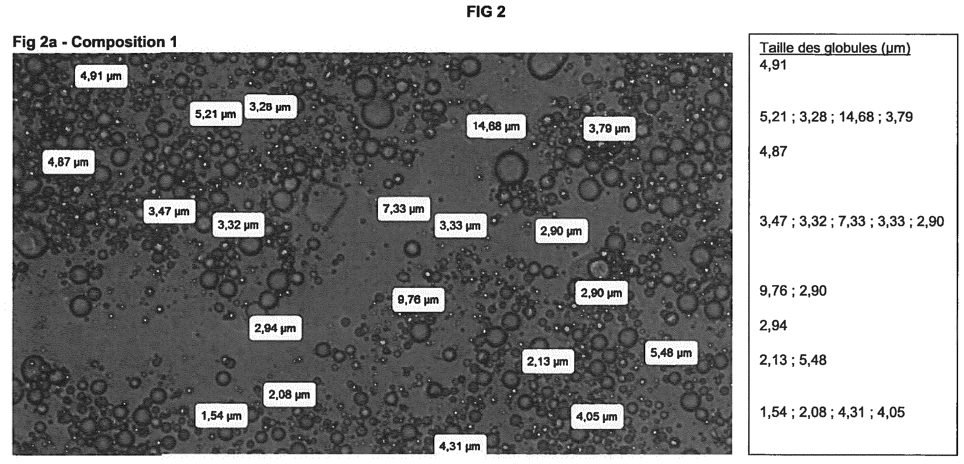

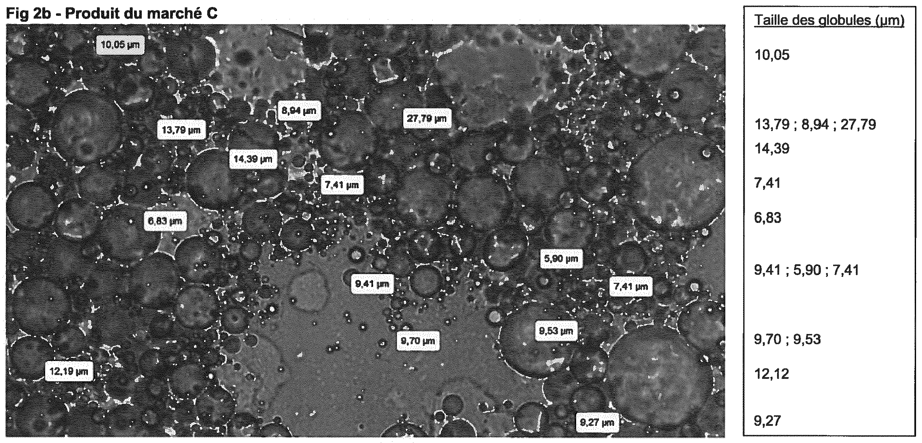

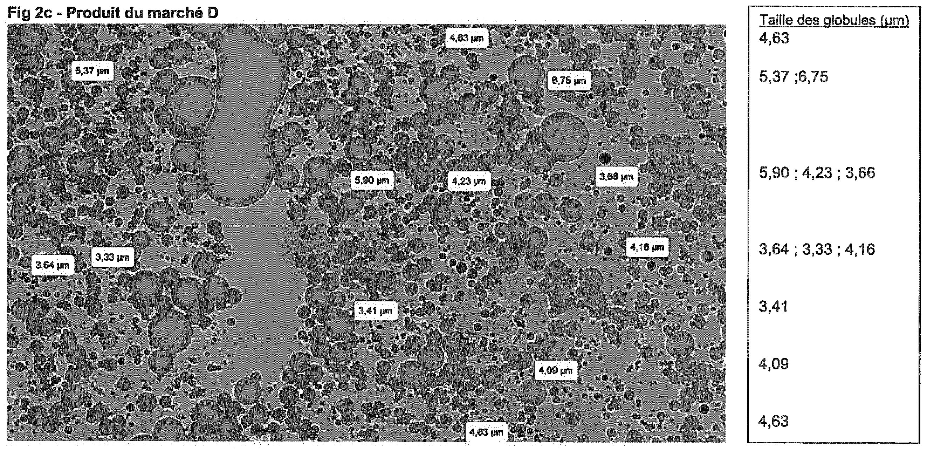

Les résultats obtenus sont illustrés à la **figure 2****.**

La composition 1 de l'invention (**fig. 2a**) présente en moyenne des globules plus petits que ceux des compositions C (**fig. 2b**) et D (**fig. 2c**) de l'art antérieur.

De plus la composition 1 présente également une meilleure homogénéité de l'aspect globulaire que celle des compositions C et D.

### Conclusion

La finesse de l'émulsion de la composition 1 entraîne une meilleure stabilité dans le temps. La formulation spécifique de la composition 1 de l'invention, de par la combinaison de cire d'abeille et du dérivé de cires végétales tel que défini ci-dessus, dans les concentrations indiquées, permettent une meilleure émulsion entre la phase aqueuse et la phase grasse et une meilleure stabilité de la formule.

### Exemple 4

Le profil rhéologique de la composition 1 de l'invention a été comparé à celui des compositions C et D de l'art antérieur, décrites dans l'exemple 2 ci-dessus.

### Appareillage utilisé :

Rhéomètre 200.
Conditions opératoires :
Système de mesure 22,
Rampe ascendante : Durée 60s, 90 points de mesure, gradient début 1s, gradient fin 250s,
Plateau : Durée 40s, gradient 250 s, vitesse constante,
Rampe descendante : Durée 60s, 90 points de mesure, gradient début 250 s, gradient fin 1s.

Mesures réalisées sur les produits portés à 32°C.

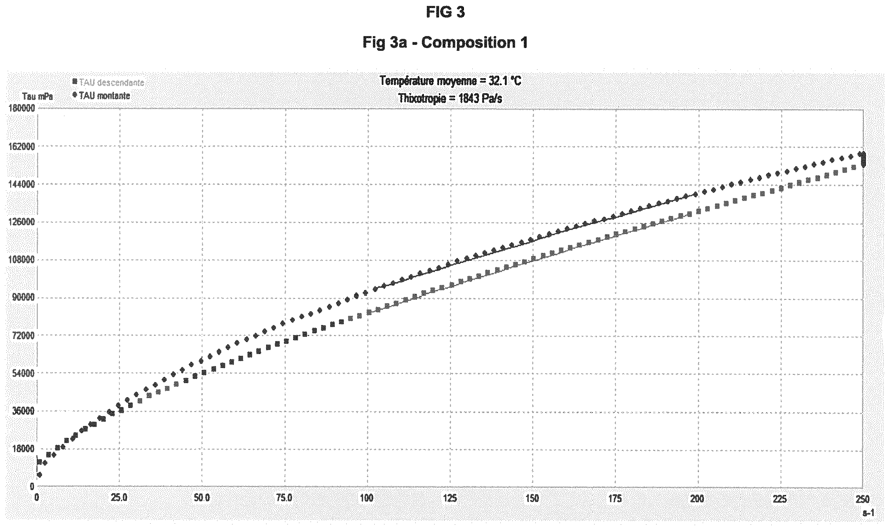

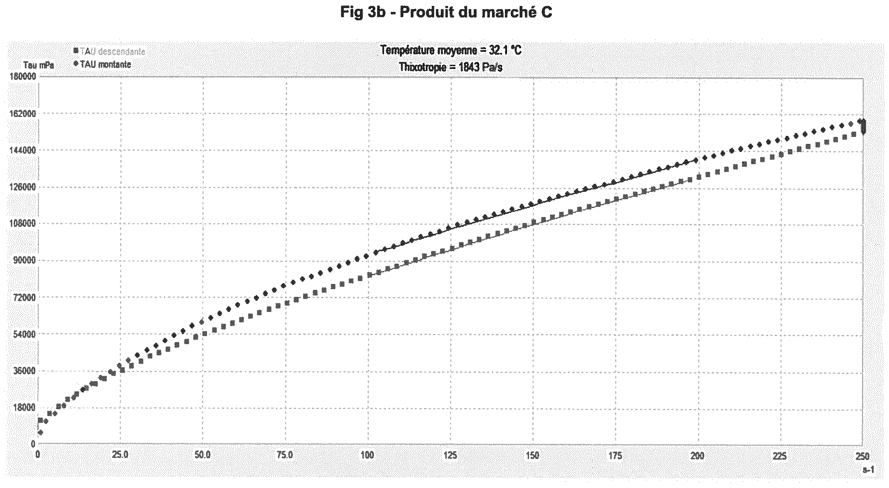

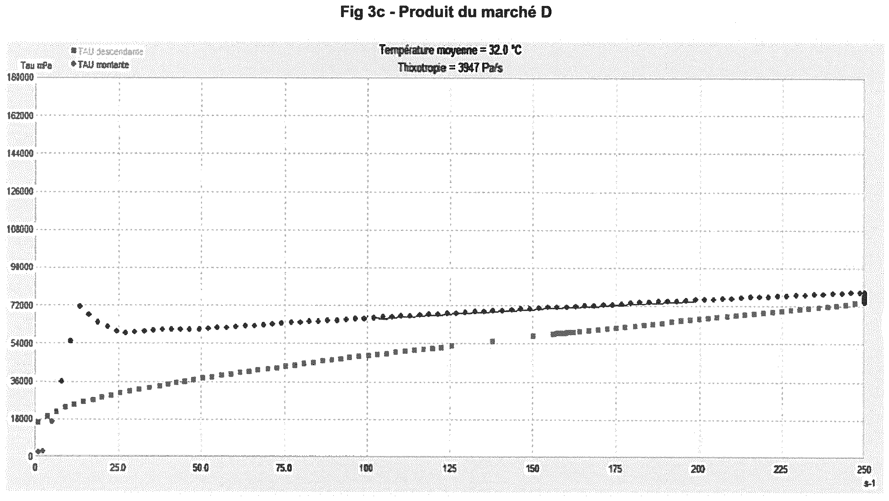

Les résultats obtenus sont illustrés à la **figure 3****.**

Les valeurs de thixotropie pour les 3 compositions sont :
Composition 1 : 1843 Pa/s (**fig. 3a**),
Composition C : 5083 Pa/s (**fig. 3b**),
Composition D : 3947 Pa/s (**fig. 3c**).

Les trois compositions présentent un profil rhéofluidifiant.

La composition 1 présente une très faible thixotropie comparée aux compositions C et D de l'art antérieur.

La composition 1 de l'invention ne casse pas ou très peu lors de l'application de celle-ci sur la peau, c'est-à-dire qu'il n'y a pas de déstructuration de la composition de l'invention.

La viscosité de la composition 1 est plus faible que celle des compositions du marché C et D, ce qui présente l'avantage de permettre une meilleure étalabilité sur la peau et la formation d'un biofilm protecteur plus uniforme.

## Revendications

1. Composition, **caractérisée en ce qu'**elle comprend :
- 40% à 54%, de préférence 44% à 50%, et plus préférentiellement encore 46% à 48% d'une huile végétale choisie dans le groupe comprenant l'huile d'olive, l'huile de macadamia, l'huile d'amande douce, l'huile de lin, l'huile d'abricot, l'huile d'argan, l'huile d'avocat, l'huile d'argousier, l'huile de germe de blé, l'huile de jojoba, l'huile de karité, l'huile d'onagre, l'huile de pépin de raisin, l'huile de noisette, l'huile de sésame, l'huile de soja, l'huile de tournesol, l'huile de bourrache et leurs mélanges, et de préférence l'huile d'olive,
- 40% à 60%, de préférence 45% à 55%, et plus préférentiellement encore 48% à 52% d'une solution d'hydroxyde de calcium,
- 0,5% à 8%, de préférence 1% à 6%, et plus préférentiellement encore 1,5% à 2,5% de cire d'abeille Cera Alba,
- 0,1 à 1,2% d'une cire végétale choisie parmi :
- un dérivé de cires obtenu par trans-estérification de la cire de jojoba, de la cire de tournesol et de la cire de mimosa, ou
- un mélange d'ester de jojoba, de cire de tournesol et de cire de mimosa,
les pourcentages étant en poids par rapport au poids total de la composition.

2. Composition selon la revendication 1, **caractérisée en ce que** l'huile végétale est de l'huile d'olive.

3. Composition selon la revendication 1, **caractérisée en ce que** l'huile végétale est un mélange d'huile d'olive et d'une autre huile végétale choisie dans le groupe comprenant l'huile de macadamia, l'huile d'amande douce, l'huile de lin, l'huile d'abricot, l'huile d'argan, l'huile d'avocat, l'huile d'argousier, l'huile de germe de blé, l'huile de jojoba, l'huile de karité, l'huile d'onagre, l'huile de pépin de raisin, l'huile de noisette, l'huile de sésame, l'huile de soja, l'huile de tournesol, l'huile de bourrache et leurs mélanges.

4. Composition selon la revendication 1 ou 3, **caractérisée en ce que** le ratio « huile d'olive/autre huile végétale » est de 99,9/0,1 à 40/60 en poids par rapport au poids total du mélange « huile d'olive et autre huile végétale ».

5. Composition selon l'une quelconque des revendications 1 à 4, **caractérisée en ce qu'**elle comprend en outre :
- un extrait de plante, notamment choisi dans le groupe comprenant un extrait de camomille, de guimauve, de mauve, d'aloe vera, d'euphraise, d'ortie, de sauge, de souci, de pivot, de pivoine, de mélèze, de pensée, de patience, de pâquerette, de pissenlit, de ronce, de souci, de saponaire, de molène, de fleur de Calendula, de plante de la famille des Malvaceae et leurs mélanges,
- un extrait de fruit, notamment choisi dans le groupe comprenant un extrait de canneberge, de cassis, de myrtille, de grenade, de framboise, de mûre, de figuier, de prunelle et leurs mélanges,
- une huile essentielle, notamment choisie dans le groupe comprenant l'huile essentielle de bergamote, de rose, d'orange douce, de lavandin, de cyprès, de niaouli, de romarin, de sauge, de géranium, de thym, d'eucalyptus globulus, de menthe, de matricaire, de genévrier, de colchique, de pivot d'Amérique, de vanille et leurs mélanges,
- de l'oxyde de zinc, du bicarbonate de soude, du silicate de magnésium et/ou du D-panthénol,
- un agent de restructuration cellulaire, de préférence l'acide hyaluronique,
- un prébiotique, notamment choisi dans le groupe comprenant des glucooligosaccharides, des fructooligosaccharides et leurs mélanges,
- un probiotique, de préférence les lactobacillus,
- un agent antimicrobien,
- un agent antifongique, et/ou
- un parfum naturel.

6. Composition selon la revendication 5, **caractérisée en ce que** :
- l'extrait de plante est présent en une quantité en poids allant de 0% à 1,5%, de préférence de 0,1% à 1%,
- l'extrait de fruit est présent en une quantité en poids allant de 0% à 2%, de préférence de 0,5% à 1,5%,
- l'extrait d'huile essentielle est présent en une quantité en poids allant de 0% à 1,5%, de préférence de 0,1% à 1%,
- l'oxyde de zinc, le bicarbonate de soude, le silicate de magnésium et/ou le D-panthénol est (sont) présent(s) en une quantité en poids allant de 0% à 5%, de préférence de 0,1% à 3%,
- l'agent de restructuration cellulaire est présent en une quantité en poids allant de 0% à 0,3%, de préférence de 0,03% à 0,1%,
- le prébiotique est présent en une quantité en poids allant de 0 à 5%, de préférence de 0,5 à 3%,
- l'agent antimicrobien et/ou l'agent antifongique est (sont) présent (s) en une quantité en poids allant de 0% à 3%, de préférence de 0,1% à 2%, et plus préférentiellement encore de 0,3 à 1%,
- le parfum est présent une quantité en poids allant de 0% à 0,5%, de préférence de 0,01% à 0,1%, et plus préférentiellement encore de 0,03 à 0,07%,
chacune desdites quantités en poids étant définie par rapport au poids total de la composition.

7. Composition selon la revendication 5, **caractérisée en ce que** le probiotique est présent en une quantité de 150 millions à 15 milliards, de préférence de 1 milliard à 5 milliards d'unités vivantes.

8. Utilisation non thérapeutique d'une composition selon l'une quelconque des revendications 1 à 7, dans une composition pour une application topique.

9. Composition selon l'une quelconque des revendications 1 à 7, **caractérisée en ce qu'**elle comprend en outre un composé choisi dans le groupe comprenant un agent émollient, un agent humectant, un agent émulsifiant, une gomme et leurs mélanges.

10. Composition selon la revendication 9, **caractérisée en ce que** :
- l'agent émollient est choisi dans le groupe comprenant le beurre, l'huile de karité, le cholestérol, le beurre de cacao, un triglycéride d'acide caprylique /caprique, et leurs mélanges,
- l'agent humectant est choisi dans le groupe comprenant le xylitol, le sorbitol, le glycérol, le pentylène glycol, le sodium hyaluronate et leurs mélanges, et est de préférence le xylitol,
- l'agent émulsifiant est choisi dans le groupe comprenant un ester de sorbitane, le monostéarate de glycérol, la lécithine de soja, un mélange d'un alcool en C₁₄₋₂₂ et d'un glucoside d'alkyle en C₁₂₋₂₀, un mélange d'un alcool cétéarylique et de cétéaryl glucoside, un mélange d'un alcool arachidylique, d'un alcool béhénylique et d'un arachydil glucoside, et leurs mélanges,
- la gomme est choisie dans le groupe comprenant la gomme d'acacia, la gomme xanthane, la gomme de guar, la gomme de biosaccharide et leurs mélanges.

11. Composition selon la revendication 9 ou la revendication 10, **caractérisée en ce que** :
- la composition selon l'une quelconque des revendications 1 à 7 est présente en une quantité en poids allant de 1% à 95%,
- le composé choisi dans le groupe comprenant un agent émollient, un agent humectant, un agent émulsifiant, une gomme et leurs mélanges est présent en une quantité en poids allant de 5% à 99%,
chacune desdites quantités en poids étant définie par rapport au poids total de la composition.

12. Utilisation non thérapeutique d'une composition selon l'une quelconque des revendications 1 à 7 ou 9 à 11, sous forme de crème, pommade, mousse, cérat ou baume.

13. Utilisation non thérapeutique d'une composition selon l'une quelconque des revendications 1 à 7 ou 9 à 11, pour nettoyer et/ou hydrater la peau.

14. Composition selon l'une quelconque des revendications 1 à 7 ou 9 à 11, pour une utilisation dans la cicatrisation, la réparation et/ou l'apaisement de la peau.

15. Composition pour une utilisation selon la revendication 14, dans laquelle la composition se trouve sous forme de crème, pommade, mousse, cérat ou baume.

## Patentansprüche

1. Zusammensetzung, **dadurch gekennzeichnet, dass** sie umfasst:
- 40 % bis 54 %, bevorzugt 44 % bis 50 %, und stärker bevorzugt 46 % bis 48 % eines Pflanzenöls, gewählt aus der Gruppe, umfassend Olivenöl, Macadamiaöl, Süßmandelöl, Leinöl, Aprikosenöl, Arganöl, Avocadoöl, Sanddornöl, Weizenkeimöl, Jojobaöl, Sheaöl, Nachtkerzenöl, Traubenkernöl, Haselnussöl, Sesamöl, Sojaöl, Sonnenblumenöl, Borretschöl und Mischungen daraus, und bevorzugt Olivenöl,
- 40 % bis 60 %, bevorzugt 45 % bis 55 %, und stärker bevorzugt 48 % bis 52 % einer Calciumhydroxid-Lösung,
- 0,5 % bis 8 %, bevorzugt 1 % bis 6 %, und noch stärker bevorzugt 1,5 % bis 2,5 % Cera Alba Bienenwachs,
- 0,1 % bis 1,2 % eines pflanzlichen Wachses, gewählt aus:
- einem Wachsderivat, erhalten durch Umesterung von Jojobawachs, Sonnenblumenwachs und Mimosenwachs, oder
- einer Mischung aus Jojobaester, Sonnenblumenwachs und Mimosenwachs, wobei die Prozentsätze Gewichtsprozente bezogen auf das Gesamtgewicht der Zusammensetzung sind.

2. Zusammensetzung nach Anspruch 1, **dadurch gekennzeichnet, dass** das Pflanzenöl Olivenöl ist.

3. Zusammensetzung nach Anspruch 1, **dadurch gekennzeichnet, dass** das Pflanzenöl eine Mischung aus Olivenöl und einem anderen Pflanzenöl ist, gewählt aus der Gruppe, umfassend Macadamiaöl, Süßmandelöl, Leinöl, Aprikosenöl, Arganöl, Avocadoöl, Sanddornöl, Weizenkeimöl, Jojobaöl, Sheaöl, Nachtkerzenöl, Traubenkernöl, Haselnussöl, Sesamöl, Sojaöl, Sonnenblumenöl, Borretschöl und Mischungen daraus.

4. Zusammensetzung nach Anspruch 1 oder 3, **dadurch gekennzeichnet, dass** das Gewichtsverhältnis "Olivenöl/anderes Pflanzenöl" 99,9/0,1 bis 40/60, bezogen auf das Gesamtgewicht der Mischung "Olivenöl und anderes Pflanzenöl", beträgt.

5. Zusammensetzung nach einem der Ansprüche 1 bis 4, **dadurch gekennzeichnet, dass** sie ferner umfasst:
- einen Pflanzenextrakt, insbesondere gewählt aus der Gruppe, umfassend einen Extrakt aus Kamille, Eibisch, Malve, Aloe Vera, Euphrasia, Brennnessel, Salbei, Ringelblume, Mohn, Pfingstrose, Lärche, Stiefmütterchen, Patientia, Gänseblümchen, Löwenzahn, Brombeere, Ringelblume, Seifenkraut, Königskerze, Calendulablüten, von Pflanzen der Familie Malvaceae und Mischungen daraus,
- einen Fruchtextrakt, insbesondere gewählt aus der Gruppe, umfassend einen Extrakt aus Preiselbeere, schwarzer Johannisbeere, Heidelbeere, Granatapfel, Himbeere, Brombeere, Feige, Schlehe und Mischungen daraus,
- ein ätherisches Öl, insbesondere gewählt aus der Gruppe, umfassend das ätherische Öl der Bergamotte, Rose, Süßorange, Lavendel, Zypresse, Niaouli, Rosmarin, Salbei, Geranie, Thymian, Eucalyptus globulus, Minze, Matricaria, Wacholder, Colchicum, Kalifornischer Mohn, Vanille und Mischungen daraus,
- Zinkoxid, Natriumbikarbonat, Magnesiumsilikat und/oder D-Panthenol,
- ein Zellrestrukturierungsmittel, bevorzugt Hyaluronsäure,
- ein Präbiotikum, insbesondere gewählt aus der Gruppe, umfassend Glucooligosaccharide, Fructooligosaccharide und Mischungen daraus,
- ein Probiotikum, bevorzugt Lactobacillus,
- ein antimikrobielles Mittel,
- ein Antimykotikum, und/oder
- einen natürlichen Duftstoff.

6. Zusammensetzung nach Anspruch 5, **dadurch gekennzeichnet, dass**
- der Pflanzenextrakt in einer Gewichtsmenge von 0 bis 1,5 %, bevorzugt von 0,1 bis 1 % vorliegt,
- der Fruchtextrakt in einer Gewichtsmenge von 0 bis 2 %, bevorzugt von 0,5 bis 1,5 %, vorliegt,
- der Extrakt des ätherischen Öls in einer Gewichtsmenge von 0 bis 1,5 %, bevorzugt von 0,1 bis 1 %, vorliegt,
- Zinkoxid, Natriumbicarbonat, Magnesiumsilikat und/oder D-Panthenol in einer Gewichtsmenge von 0 bis 5 %, bevorzugt von 0,1 bis 3 %, vorliegen,
- das Zellrestrukturierungsmittel in einer Gewichtsmenge von 0 bis 0,3 %, bevorzugt 0,03 % bis 0,1 %, vorliegt,
- das Präbiotikum in einer Gewichtsmenge von 0 bis 5 %, bevorzugt von 0,5 bis 3 %, vorliegt,
- das antimikrobielle Mittel und/oder das Antimykotikum in einer Gewichtsmenge von 0 bis 3 %, bevorzugt von 0,1 % bis 2 % und noch stärker bevorzugt von 0,3 % bis 1 % vorliegen,
- der Duftstoff in einer Gewichtsmenge im Bereich von 0 bis 0,5 %, bevorzugt von 0,01 bis 0,1 %, und noch stärker bevorzugt von 0,03 bis 0,07 %, vorliegt,
wobei jede der Gewichtsmengen in Bezug auf das Gesamtgewicht der Zusammensetzung definiert ist.

7. Zusammensetzung nach Anspruch 5, **dadurch gekennzeichnet, dass** das Probiotikum in einer Menge von 150 Millionen bis 15 Milliarden, bevorzugt von einer Milliarde bis fünf Milliarden lebende Einheiten, vorliegt.

8. Nicht-therapeutische Verwendung einer Zusammensetzung nach einem der Ansprüche 1 bis 7 in einer Zusammensetzung zur topischen Anwendung.

9. Zusammensetzung nach einem der Ansprüche 1 bis 7, **dadurch gekennzeichnet, dass** sie ferner eine Verbindung umfasst, die aus der Gruppe gewählt ist, umfassend einen Weichmacher, ein Feuchthaltemittel, einen Emulgator, Gummi und Mischungen daraus.

10. Zusammensetzung nach Anspruch 9, **dadurch gekennzeichnet, dass**
- der Weichmacher gewählt ist aus der Gruppe, umfassend Butter, Sheaöl, Cholesterin, Kakaobutter, ein Capryl-/Caprinsäuretriglycerid und Mischungen daraus,
- das Feuchthaltemittel gewählt ist aus der Gruppe, umfassend Xylit, Sorbit, Glycerin, Pentylenglykol, Natriumhyaluronat und Mischungen daraus, und bevorzugt Xylit ist,
- der Emulgator gewählt ist aus der Gruppe, umfassend einen Sorbitanester, Glycerinmonostearat, Sojalecithin, eine Mischung aus einem C₁₄₋₂₂-Alkohol und einem C₁₂₋₂₀-Alkylglucosid, eine Mischung aus einem Cetearylalkohol und Cetearylglucosid, eine Mischung aus einem Arachydilalkohol, einem Behenylalkohol und einem Arachydilglucosid und Mischungen daraus,
- das Gummi aus der Gruppe gewählt ist, umfassend Akaziengummi, Xanthangummi, Guargummi, Biosaccharidgummi und Mischungen daraus.

11. Zusammensetzung nach Anspruch 9 oder Anspruch 10, **dadurch gekennzeichnet, dass**
- die Zusammensetzung nach einem der Ansprüche 1 bis 7 in einer Gewichtsmenge im Bereich von 1 bis 95 % vorliegt,
- die aus der Gruppe, umfassend einen Weichmacher, ein Feuchthaltemittel, einen Emulgator, Gummi und Mischungen daraus, gewählte Verbindung in einer Gewichtsmenge im Bereich von 5 bis 99 % vorliegt,
wobei jede der Gewichtsmengen in Bezug auf das Gesamtgewicht der Zusammensetzung definiert ist.

12. Nicht-therapeutische Verwendung einer Zusammensetzung nach einem der Ansprüche 1 bis 7 oder 9 bis 11 in Form einer Creme, Salbe, eines Schaums, einer Wachscreme oder eines Balsams.

13. Nicht-therapeutische Verwendung einer Zusammensetzung nach einem der Ansprüche 1 bis 7 oder 9 bis 11 zur Reinigung und/oder Befeuchtung der Haut.

14. Zusammensetzung nach einem der Ansprüche 1 bis 7 oder 9 bis 11 zur Verwendung bei der Heilung, Reparatur und/oder Beruhigung der Haut.

15. Zusammensetzung für eine Verwendung nach Anspruch 14, wobei die Zusammensetzung in Form einer Creme, Salbe, eines Schaums, einer Wachscreme oder eines Balsams vorliegt.

## Claims

1. Composition, **characterised in that** it comprises:
- 40% to 54%, preferably 44% to 50%, and even more preferably 46% to 48% of a vegetable oil chosen from the group comprising olive oil, macadamia oil, sweet almond oil, linseed oil, apricot oil, argan oil, avocado oil, sea buckthorn oil, wheat germ oil, jojoba oil, shea oil, evening primrose oil, grape seed oil, hazelnut oil, sesame oil, soybean oil, sunflower oil, borage oil and mixtures thereof, preferably olive oil,
- 40% to 60%, preferably 45% to 55%, and even more preferably 48% to 52% of a solution of calcium hydroxide,
- 0.5% to 8%, preferably 1% to 6%, and even more preferably 1.5% to 2.5% of Cera Alba beeswax,
- 0.1 to 1.2% of a vegetable wax chosen from:
-- a derivative of waxes, obtained by transesterification of jojoba wax, of sunflower wax and of mimosa wax, or
-- a mixture of jojoba ester, sunflower wax and mimosa wax,
the percentages being by weight based on the total weight of the composition.

2. Composition according to claim 1, **characterised in that** the vegetable oil is olive oil.

3. Composition according to claim 1, **characterised in that** the vegetable oil is a mixture of olive oil and of another vegetable oil chosen from the group comprising macadamia oil, sweet almond oil, linseed oil, apricot oil, argan oil, avocado oil, sea buckthorn oil, wheat germ oil, jojoba oil, shea oil, evening primrose oil, grape seed oil, hazelnut oil, sesame oil, soybean oil, sunflower oil, borage oil and mixtures thereof.

4. Composition according to claim 1 or 3, **characterised in that** the ratio "olive oil/other vegetable oil" is from 99.9/0.1 to 40/60 by weight based on the total weight of the mixture "olive oil and other vegetable oil".

5. Composition according to any one of claims 1 to 4, **characterised in that** it further comprises:
- a plant extract, in particular chosen from the group comprising an extract of camomile, of marsh-mallow, of mallow, of aloe vera, of eyebright, of nettle, of sage, of marigold, of taproot, of peony, of larch, of pansy, of dock, of daisy, of dandelion, of blackberry, of marigold, of soapwort, of mullein, of *Calendula* flower, of a plant of the Malvaceae family and mixtures thereof,
- a fruit extract, in particular chosen from the group comprising an extract of cranberry, of black currant, of blueberry, of pomegranate, of raspberry, of blackberry, of fig tree, of sloe and mixtures thereof,
- an essential oil, in particular chosen from the group comprising the essential oil of bergamot, of rose, of sweet orange, of lavandin, of cypress, of broad-leaved paperbark, of rosemary, of sage, of geranium, of thyme, of eucalyptus globulus, of mint, of *Matricaria*, of juniper, of *Cholchicum*, of American taproot, of vanilla and mixtures thereof,
- zinc oxide, sodium bicarbonate, magnesium silicate and/or D-panthenol,
- a cellular restructuring agent, preferably hyaluronic acid,
- a prebiotic, in particular chosen from the group comprising glucooligosaccharides, fructooligosaccharides and mixtures thereof,
- a probiotic, preferably the lactobacilli,
- an antimicrobial agent,
- an antifungal agent, and/or
- a natural perfume.

6. Composition according to claim 5, **characterised in that**:
- the plant extract is present in a quantity by weight ranging from 0% to 1.5%, preferably from 0.1% to 1%,
- the fruit extract is present in a quantity by weight ranging from 0% to 2%, preferably from 0.5% to 1.5%,
- the extract of essential oil is present in a quantity by weight ranging from 0% to 1.5%, preferably from 0.1% to 1%,
- the zinc oxide, the sodium bicarbonate, the magnesium silicate and/or the D-panthenol is(are) present in a quantity by weight ranging from 0% to 5%, preferably from 0.1% to 3%,
- the cellular restructuring agent is present in a quantity by weight ranging from 0% to 0.3%, preferably from 0.03% to 0.1%,
- the prebiotic is present in a quantity by weight ranging from 0 to 5%, preferably from 0.5 to 3%,
- the antimicrobial and/or antifungal agent is(are) present in a quantity by weight ranging from 0% to 3%, preferably from 0.1% to 2%, and even more preferably from 0.3 to 1%,
- the perfume is present in a quantity by weight ranging from 0% to 0.5%, preferably from 0.01% to 0.1%, and even more preferably from 0.03 to 0.07%,
each of said quantities by weight being defined based on the total weight of the composition.

7. Composition according to claim 5, **characterised in that** the probiotic is present in a quantity of 150 million to 15 billion, preferably 1 billion to 5 billion living units.

8. Non-therapeutic use of a composition according to any one of claims 1 to 7, in a composition for topical use.

9. Composition according to any one of claims 1 to 7, **characterised in that** it further comprises a compound chosen from the group comprising an emollient agent, a moistening agent, an emulsifying agent, a gum and mixtures thereof.

10. Composition according to claim 9, **characterised in that**:
- the emollient agent is chosen from the group comprising butter, shea oil, cholesterol, cocoa butter, a triglyceride of caprylic/capric acid, and mixtures thereof,
- the moistening agent is chosen from the group comprising xylitol, sorbitol, glycerol, pentylene glycol, sodium hyaluronate and mixtures thereof, and is preferably xylitol,
- the emulsifying agent is chosen from the group comprising an ester of sorbitan, glycerol monostearate, soy lecithin, a mixture of a C₁₄₋₂₂ alcohol and a C₁₂₋₂₀ alkyl glucoside, a mixture of a cetearyl alcohol and a cetearyl glucoside, a mixture of an arachidyl alcohol, of a behenyl alcohol and of an arachydil glucoside, and mixtures thereof,
- the gum is chosen from the group comprising acacia gum, xanthan gum, guar gum, biosaccharide gum and mixtures thereof.

11. Composition according to claim 9 or claim 10, **characterised in that**:
- the composition according to any one of claims 1 to 7 is present in a quantity by weight ranging from 1% to 95%,
- the compound chosen from the group comprising an emollient agent, a moistening agent, an emulsifying agent, a gum and mixtures thereof is present in a quantity by weight ranging from 5% to 99%,
each of said quantities by weight being defined based on the total weight of the composition.

12. Non-therapeutic use of a composition according to any one of claims 1 to 7 or 9 to 11, in the form of a cream, pomade, foam, cerate or balm.

13. Non-therapeutic use of a composition according to any one of claims 1 to 7 or 9 to 11, for cleaning and/or hydrating the skin.

14. Composition according to any one of claims 1 to 7 or 9 to 11, for a use in the healing, the reparation and/or the soothing of the skin.

15. Composition for a use according to claim 14, wherein the composition is in the form of a cream, pomade, foam, cerate or balm.
